# FASCICULE DE BREVET EUROPEEN

(11) **EP 2 179 734 B1**
(45) Date de publication et mention de la délivrance du brevet: **26.02.2014**
(21) Numéro de dépôt: 09306018.4
(22) Date de dépôt: 27.10.2009
(51) Int. Cl.: A61K 31/517, A61K 31/536, A61P 25/32

(54) **Dérivés de la benzoxazine et de la dihydroquinazoline pour leur utilisation dans le traitement du syndrome de sevrage alcoolique**
Benzoxazin- und Dihydrochinazolinderivate zur Verwendung in der Behandlung von Alkoholentzugssyndrom
Benzoxazine and dihydroquinazoline derivatives for use in the treatment of alcohol withdrawal syndrome

(30) Priorité: 27.10.2008 FR 0857277
(43) Date de publication de la demande: 28.04.2010
(73) Titulaire: Biocodex, 94250 Gentilly (FR)
(72) Inventeur: Verleye, Marc, 60190, REMY (FR); Le Guern, Marie-Emmanuelle, 60200, COMPIEGNE (FR); Gillardin, Jean-Marie, 60680, JONQUIERES (FR); Hublot, Bernard, 60200, COMPIEGNE (FR)
(74) Mandataire: Vial, Lionel

(56) Documents cités:
- EP-A1- 1 745 785
- EP-A1- 1 745 786
- WO-A1-01/13921
- WO-A2-2006/098969
- WO-A2-2007/109288
- SHARMA, A.N.; UGALE, R.R.; CHOPDE, C.T. (INDIAN J PHARMACOL 2005, 37(SUPPL.): S22): "Reversal of ethanol withdrawal anxiety by non-benzodiazepine anxiolytic etifoxine: Role for neurosteroid allopregnanolone" BIOMEDICAL LITERATURE REFERENCE FROM PROUS SCIENCE INTEGRITY., [Online] 2005, XP002534089 Extrait de l'Internet: URL:http://integrity.prous.com/integrity/s ervlet/xmlxsl/pk_ref_list.xml_show_llistat _refs?>
- SHARMA ET AL: "Chronic progesterone treatment augments while dehydroepiandrosterone sulphate prevents tolerance to ethanol anxiolysis and withdrawal anxiety in rats" EUROPEAN JOURNAL OF PHARMACOLOGY, ELSEVIER BV, NL, vol. 567, no. 3, 27 juin 2007 (2007-06-27) , pages 211-222, XP022132884 ISSN: 0014-2999
- VERLEYE M ET AL: "The anxiolytic etifoxine protects against convulsant and anxiogenic aspects of the alcohol withdrawal syndrome in mice" ALCOHOL, PERGAMON PRESS, LONDON, GB, vol. 43, no. 3, 1 mai 2009 (2009-05-01), pages 197-206, XP026021430 ISSN: 0741-8329 [extrait le 2009-04-23]
- MARC VERLEYE ET AL: "Effects of etifoxine on ligand binding to GABAA receptors in rodents" NEUROSCIENCE RESEARCH, ELSEVIER, SHANNON, IR, vol. 44, 1 janvier 2002 (2002-01-01), pages 167-172, XP007908404 ISSN: 0168-0102
- UGALE ET AL: "Neurosteroid allopregnanolone mediates anxiolytic effect of etifoxine in rats", BRAIN RESEARCH, ELSEVIER, AMSTERDAM, NL, vol. 1184, 27 November 2007 (2007-11-27), pages 193-201, XP022364574, ISSN: 0006-8993, DOI: 10.1016/J.BRAINRES.2007.09.041
- CATHERINE PARÉ ET AL: 'Le traitement du syndrome de sevrage de l'alcool' PHARMACTUEL, [en ligne] 01 Janvier 2007, pages 25 - 32, XP055019839 Extrait de l'Internet: <URL:http://www.pharmactuel.com/sommaires/2 00701pt.pdf> [extrait le 2012-02-20]

## Description

### Domaine de l'invention

La présente invention concerne une composition pharmaceutique destinée à la prévention ou au traitement du syndrome de sevrage alcoolique.

### Arrière plan technique

A l'heure actuelle, la consommation inadaptée d'alcool est encore la cause directe ou indirecte de plusieurs dizaines de milliers de décès par an et de plus de 15% des hospitalisations en France. Parmi les personnes les plus à risque se trouvent les alcoolodépendants, qui ne peuvent contrôler leur consommation d'alcool, et dont la prise en charge thérapeutique nécessite un sevrage et une abstinence durable. Les objectifs, les indications et les modalités du sevrage du patient alcoolodépendant ont notamment fait l'objet d'une conférence de consensus, à l'initiative de l'Agence Nationale d'Accréditation et d'Evaluation en Santé et de la Société Française d'Alcoologie, le 17 mars 1999, qui a abouti à un texte de recommandations (voir également Couzigou et Ledinghen (2002) Gastroenterol. Clin. Biol. 26 :B163-B168).

Ainsi, on définit le sevrage alcoolique comme étant un arrêt de la consommation d'alcool, qu'il soit accidentel, à l'initiative du sujet ou qu'il s'inscrive dans une perspective thérapeutique (sevrage thérapeutique), chez l'alcoolodépendant. Le sevrage alcoolique est notamment décrit dans le paragraphe 291.8 "Sevrage alcoolique" de la quatrième version du « Diagnostic and Statistical Manual » (DSM IV) éditée par l'American Psychiatric Association en 1994.

On observe souvent des manifestations symptomatiques survenant dans les suites immédiates ou différées jusqu'au dixième jour suivant cet arrêt, qui traduisent un état de manque psychique, comportemental et physique. Ces manifestations sont constitutives du syndrome de sevrage alcoolique.

Plus particulièrement, le syndrome de sevrage associe de façon variable plusieurs types de manifestations. On observe dans la grande majorité des cas :
- des troubles subjectifs : anxiété, agitation, irritabilité, insomnies, cauchemars ;
- des troubles neurovégétatifs : sueurs, tremblements, tachycardie, hypertension artérielle ;
- des troubles digestifs : anorexie, nausées, vomissements.

Dans les heures qui suivent, ce tableau peut s'aggraver, ou se compliquer, par l'apparition :
- de signes confusionnels : troubles de la concentration, de la mémoire, du jugement ;
- d'hallucinations ;
- de delirium, notamment de delirium tremens ;
- de convulsions ;
- d'hyperthermie.

Le tableau clinique le plus caractéristique et le plus sévère est constitué par le delirium tremens.

Ce syndrome peut faire l'objet d'un traitement médicamenteux. A ce titre, les benzodiazépines (BZD) sont le traitement de première intention du syndrome de sevrage alcoolique, dans la mesure où c'est pour cette classe de médicaments que l'effet thérapeutique a été le mieux démontré. Les BZD réduisent ainsi l'incidence et la sévérité du syndrome de sevrage. Le diazépam (Valium®) est la BZD la plus prescrite pour cette indication, mais l'oxazépam, le lorazépam, l'alprazolam et le chlorazépate peuvent également être prescrits.

Toutefois, les BZD sont à l'origine de manifestations indésirables bien connues, telles qu'une hypotonie des muscles de la gorge, une dépression respiratoire, une somnolence, ou encore des troubles de la mémoire. En outre, il existe également un risque non négligeable de dépendance aux BZD conduisant à limiter la durée de traitement par ces composés. Enfin, la consommation concomitante d'alcool et de BZD est susceptible d'engendrer de graves troubles du comportement tels que violences et actes automatiques amnésiques, impliquant un arrêt strict de la consommation d'alcool, ce qui peut être difficile à obtenir d'un alcoolodépendant, en l'absence de surveillance.

Ainsi, l'objet de la présente invention est de fournir de nouveaux composés, dépourvus des inconvénients des composés déjà connus, notamment les BZD, mais présentant une efficacité au moins semblable, dans le cadre de la prévention ou du traitement du syndrome de sevrage alcoolique.

L'étifoxine, ou chlorhydrate de 6-chloro-2-éthylamino-4-méthyl-4-phényl-4H-[3,1]benzoxazine appartient à la famille des benzoxazines. Elle favorise la transmission gabaergique en se liant sur un site proche du canal chlore couplé au récepteur GABA_{A} et est actuellement utilisée comme anxiolytique. Peu de manifestations indésirables consécutives à son utilisation sont répertoriées.

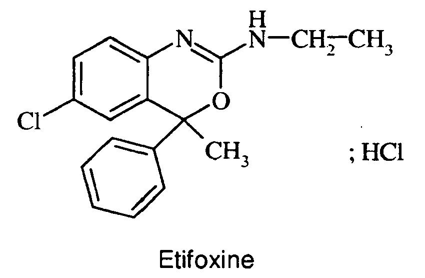

La synthèse de ce composé est notamment décrite dans le brevet français n° 1 571 287. Par ailleurs, plusieurs métabolites actifs de l'étifoxine ont été décrits, tels que la déséthyl-étifoxine ou 2-amino-6-chloro-4-méthyl-4-phényl-4H-[3,1]benzoxazine, la 6-chloro-4-(4-hydroxyphényl)-4-méthyl-3,4-dihydro-1H-quinazolin-2-one ou la 6-chloro-3-éthyl-7-hydroxy-4-méthyl-4-phényl-3,4-dihydro-1H-quinazolin-2-one.

### Résumé de l'invention

La présente invention découle de la mise en évidence inattendue, par les inventeurs, que l'étifoxine permet de prévenir ou de traiter des symptômes du sevrage alcoolique, avec une efficacité au moins semblable à celle du diazépam, dans un modèle animal.

En conséquence, la présente invention concerne un composé de formule (I) suivante : 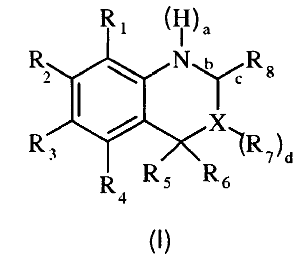 dans laquelle :
- a représente 0 ou 1 ;
- b représente une simple liaison ou une double liaison ;
- c représente une simple liaison ou une double liaison ;
- d représente 0 ou 1 ;
- X représente un atome d'oxygène ou d'azote, sous réserve que lorsque X représente un atome d'oxygène alors d vaut 0 et que lorsque X représente un atome d'azote alors d vaut 1 ;
- R₁, R₂, R₃, et R₄, identiques ou différents, représentent un atome d'hydrogène, un atome d'halogène, notamment choisi parmi F, CI, Br, ou I, un groupe hydroxyle, ou un groupe alkoxyle de 1 ou 2 atomes de carbone ;
- R₅ et R₆, identiques ou différents, représentent un atome d'hydrogène, un groupe alkyle ou cycloalkyle de 1 à 6 atomes de carbone, ou un groupe aryle de 6 atomes de carbone dont le noyau aromatique est éventuellement substitué par un ou plusieurs atomes d'halogène ou un ou plusieurs groupes hydroxyle, alkoxyle de 1 ou 2 atomes de carbone, trifluorométhyle ou nitro ;
- R₇ représente un atome d'hydrogène, un groupe hydroxyle, ou un groupe alkyle ou hydroxyalkyle de 1 à 3 atomes de carbone ;
- R₈ représente un atome d'oxygène ou un groupe -NR₉R₁₀, R₉ et R₁₀, identiques ou différents, représentant un atome d'hydrogène, un groupe hydroxyle, ou un groupe alkyle ou hydroxyalkyle de 1 à 3 atomes de carbone, sous réserve que lorsque R₈ représente un atome d'oxygène alors a vaut 1, b représente une simple liaison et c représente une double liaison et que lorsque R₈ représente un groupe -NR₉R₁₀ alors a vaut 0, b représente une double liaison et c représente une simple liaison ;
ou un sel pharmaceutiquement acceptable de ce composé,
pour son utilisation dans la prévention ou le traitement du syndrome de sevrage alcoolique.

La présente invention concerne également une utilisation dans une méthode de prévention ou de traitement du syndrome de sevrage alcoolique chez un individu, dans laquelle on administre à l'individu une quantité prophylactiquement ou thérapeutiquement efficace d'un composé de formule (I) telle que définie ci-dessus, ou d'un sel pharmaceutiquement acceptable de celui-ci.

La présente invention concerne également une composition pharmaceutique, comprenant à titre de substances actives, au moins un composé de formule (I) telle que définie ci-dessus, ou un sel pharmaceutiquement acceptable de celui-ci, et au moins un composé additionnel destiné à la prévention ou au traitement du syndrome de sevrage alcoolique, à l'exception d'une benzodiazépine, en association avec un véhicule pharmaceutiquement acceptable.

La présente invention concerne également des produits contenant :
- au moins un composé de formule (I) tel que défini ci-dessus, ou un sel pharmaceutiquement acceptable de celui-ci, et
- au moins un composé additionnel destiné à la prévention ou au traitement du syndrome de sevrage alcoolique,
comme produit de combinaison pour une utilisation simultanée, séparée ou étalée dans le temps pour la prévention ou le traitement du syndrome de sevrage alcoolique.

### Description détaillée de l'invention

### Pathologie

Dans le cadre de l'invention, la prévention ou le traitement du syndrome de sevrage alcoolique s'entend en particulier de la prévention ou du traitement d'au moins un moins un, de préférence plusieurs, symptômes de sevrage alcoolique, notamment choisis parmi des troubles subjectifs tels que l'anxiété, l'agitation, l'irritabilité, les insomnies, les cauchemars ; des troubles neurovégétatifs, tels que des sueurs, des tremblements, une tachycardie, l'hypertension artérielle ; des troubles digestifs, tels qu'une anorexie, des nausées, des vomissements ; des signes confusionnels, tels que des troubles de la concentration, de la mémoire, du jugement ; des hallucinations ; un delirium ; des convulsions. En particulier, le delirium tremens est le tableau le plus caractéristique et le plus sévère des symptômes de sevrage alcoolique.

Plus particulièrement, les composés de formule (I) telle que définie ci-dessus, ou leurs sels pharmaceutiquement acceptables, permettent de traiter l'anxiété et/ou les convulsions liées à un sevrage alcoolique.

Ainsi, la présente invention concerne en particulier les composés de formule (I) telle que définie ci-dessus, ou leurs sels pharmaceutiquement acceptables, pour leur utilisation dans la prévention ou le traitement de l'anxiété et/ou des convulsions liées à un sevrage alcoolique, et plus particulièrement pour leur utilisation dans la prévention ou le traitement des convulsions, et éventuellement de l'anxiété, liées à un sevrage alcoolique.

De la même façon, la présente invention concerne en particulier une utilisation dans une méthode de prévention ou de traitement de l'anxiété et/ou des convulsions liées à un sevrage alcoolique, et plus particulièrement une méthode de prévention ou de traitement des convulsions, et éventuellement de l'anxiété, liées à un sevrage alcoolique, chez un individu, dans laquelle on administre à l'individu une quantité prophylactiquement ou thérapeutiquement efficace d'un composé de formule (I) telle que définie ci-dessus, ou d'un sel pharmaceutiquement acceptable de celui-ci.

De préférence, l'individu chez lequel on souhaite prévenir ou traiter le syndrome de sevrage alcoolique selon l'invention est alcoolodépendant. L'alcoolodépendance est notamment définie dans le paragraphe 303.90 "Dépendance alcoolique" de la quatrième version du « Diagnostic and Statistical Manual » (DSM IV) éditée par l'American Psychiatric Association en 1994.

### Composé de formule (I)

La synthèse des composés de formule (I) définie ci-dessus peut aisément être mise en oeuvre à partir des enseignements du brevet français n° 1 571 287.

Les sels pharmaceutiquement acceptables selon l'invention apparaîtront de manière évidente pour l'homme du métier. En particulier les sels de chlorhydrate des composés de formule (I) selon l'invention sont préférés.

Comme on l'entend ici, la formule (I) définie ci-dessus englobe notamment les formules des composés de formule (I) optiquement actifs, tels que les énantiomères représentés par les formules suivantes (lorsque R₅ et R₆ sont différents) : 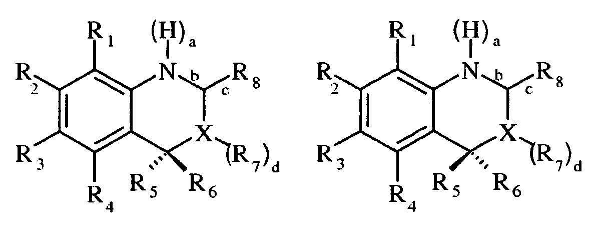 ou leurs mélanges, en particulier leur mélange racémique.

Dans un mode de réalisation préférentiel de l'invention, la formule (I) définie ci-dessus est représentée par la formule (VIII) suivante : 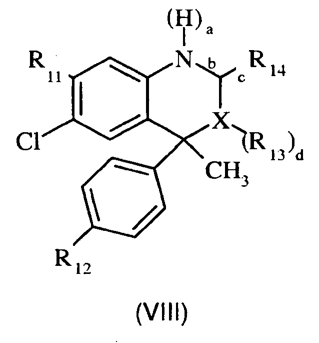 dans laquelle :
- a représente 0 ou 1 ;
- b représente une simple liaison ou une double liaison ;
- c représente une simple liaison ou une double liaison ;
- d représente 0 ou 1 ;
- X représente un atome d'oxygène ou d'azote, sous réserve que lorsque X représente un atome d'oxygène alors d vaut 0 et que lorsque X représente un atome d'azote alors d vaut 1 ;
- R₁₁ et R₁₂ identiques ou différents représentent -H ou -OH ;
- R₁₃ représente -H ou un groupe -CH₂-CH₃ ;
- R₁₄ représente un atome d'oxygène ou un groupe -NH₂ ou -NH-CH₂-CH₃, sous réserve que lorsque R₁₄ représente un atome d'oxygène alors a vaut 1, b représente une simple liaison et c représente une double liaison et que lorsque R₁₄ représente un groupe -NH₂ ou -NH-CH₂-CH₃ alors a vaut 0, b représente une double liaison et c représente une simple liaison.

Comme on l'entend ici, la formule (VIII) définie ci-dessus englobe notamment les formules des composés de formule (VIII) optiquement actifs, tels que les énantiomères représentés par les formules suivantes : 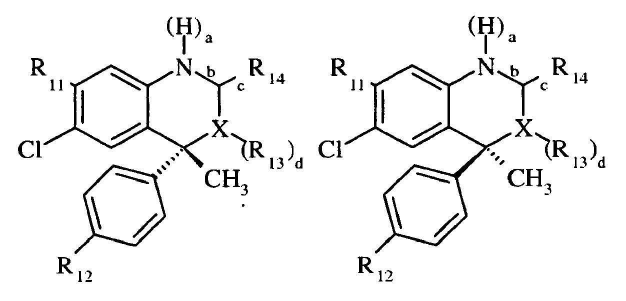 ou leurs mélanges, en particulier leur mélange racémique.

Dans un autre mode de réalisation préférentiel de l'invention, la formule (I) définie ci-dessus est représentée par la formule (II) suivante : 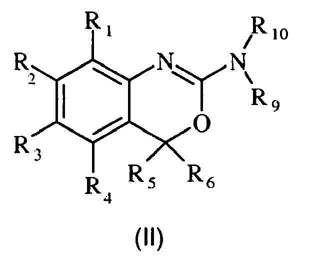 dans laquelle R₁, R₂, R₃, R₄, R₅, R₆, R₉ et R₁₀ sont tels que définis ci-dessus.

Comme on l'entend ici, la formule (II) définie ci-dessus englobe notamment les formules des composés de formule (II) optiquement actifs, tels que les énantiomères représentés par les formules suivantes (lorsque R₅ et R₆ sont différents) : 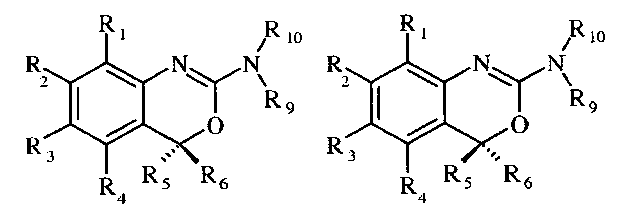 ou leurs mélanges, en particulier leur mélange racémique.

Dans un autre mode de réalisation préférentiel de l'invention, la formule (I) définie ci-dessus est représentée par les formules (III) et (IV) suivantes : 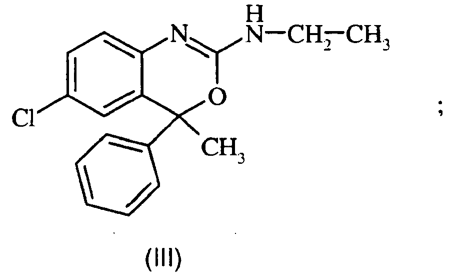 HCl

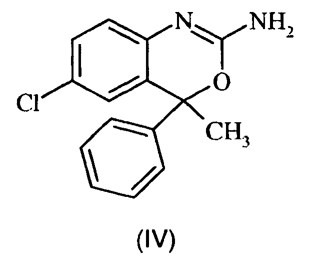

Le composé de formule (III) est l'étifoxine, ou chlorhydrate de 6-chloro-2-éthylamino-4-méthyl-4-phényl-4H-[3,1 ]benzoxazine.

Le composé de formule (IV), la déséthyl-étifoxine ou 2-amino-6-chloro-4-méthyl-4-phényl-4H-[3,1]benzoxazine, est un métabolite de l'étifoxine.

Comme on l'entend ici, la formule (III) définie ci-dessus englobe notamment les formules des composés de formule (III) optiquement actifs, tels que les énantiomères représentés par les formules suivantes : 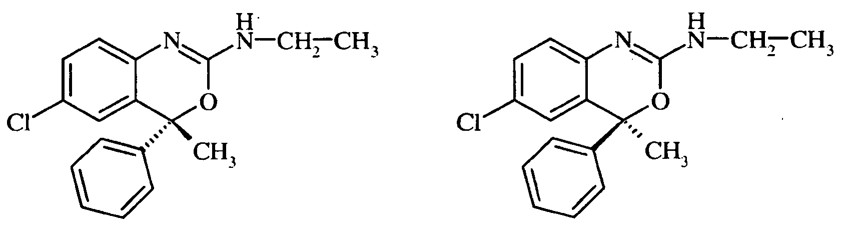 ou leurs mélanges, en particulier leur mélange racémique, notamment sous forme chlorhydrate, ainsi que les formules des composés de formule (IV) optiquement actifs, tels que les énantiomères représentés par les formules suivantes : 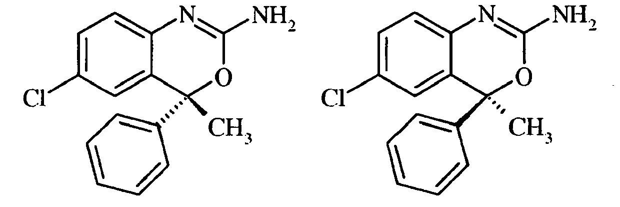 ou leurs mélanges, en particulier leur mélange racémique.

Dans un autre mode de réalisation préférentiel de l'invention, la formule (I) est représentée par la formule (V) suivante : 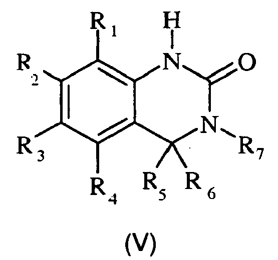 dans laquelle R₁, R₂, R₃, R₅, R₆, et R₇ sont tels que définis ci-dessus.

Comme on l'entend ici, la formule (V) définie ci-dessus englobe notamment les formules des composés de formule (V) optiquement actifs, tels que les énantiomères représentés par les formules suivantes (lorsque R₅ et R₆ sont différents) : 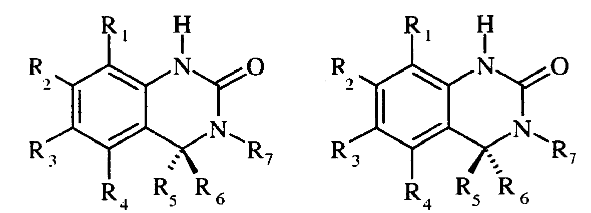 ou leurs mélanges, en particulier leur mélange racémique.

Dans un autre mode de réalisation préférentiel de l'invention, la formule (I) est représentée par les formules (VI) et (VII) suivantes :

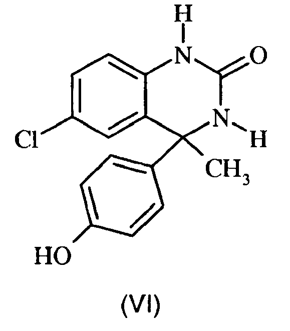

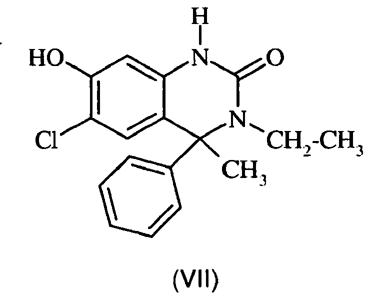

Les composés de formule (VI) (6-Chloro-4-(4-hydroxy-phényl)-4-méthyl-3,4-dihydro-1H-quinazolin-2-one) et (VII) (6-chloro-3-éthyl-7-hydroxy-4-méthyl-4-phényl-3,4-dihydro-1H-quinazolin-2-one) sont des métabolites de l'étifoxine.

Comme on l'entend ici, la formule (VI) définie ci-dessus englobe notamment les formules des composés de formule (VI) optiquement actifs, tels que les énantiomères représentés par les formules suivantes : 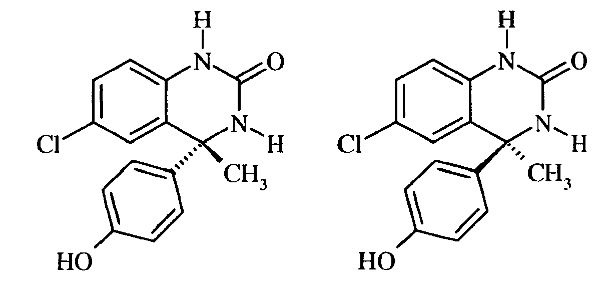 ou leurs mélanges, en particulier leur mélange racémique, ainsi que les formules des composés de formule (VII) optiquement actifs, tels que les énantiomères suivants : 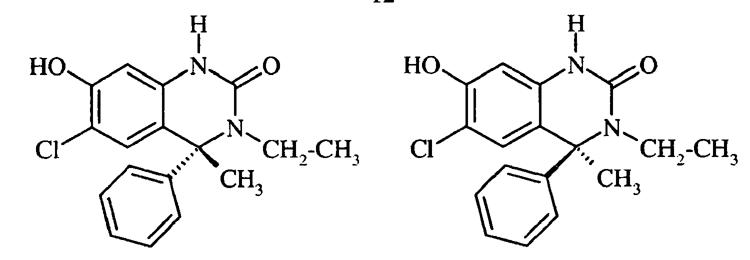 ou leurs mélanges, en particulier leur mélange racémique.

Avantageusement, les composés de formule (I) ci-dessus, ou leurs sels pharmaceutiquement acceptables, ne présentent pas de similarité structurale avec les BZD, et sont susceptibles d'agir sur un site allostérique du récepteur GABA-A différents de celui des BZD, en conséquence ils sont dépourvus de leurs effets néfastes.

### Administration

De préférence, le composé de formule (I) telle que définie ci-dessus, ou son sel pharmaceutiquement acceptable, est administré ou administrable à une dose unitaire d'environ 50 mg à environ 1500 mg, notamment d'environ 150 à environ 200 mg. De préférence également, le composé de formule (I) telle que définie ci-dessus, ou son sel pharmaceutiquement acceptable est administré ou administrable avec un régime de dosage de 50 mg/j à environ 1500 mg/j, notamment d'environ 150 mg/j à environ 200 mg/j.

De préférence, le composé de formule (I) telle que définie ci-dessus, ou son sel pharmaceutiquement est administré ou administrable sous une forme convenant pour une administration par voie orale. Plus préférablement, le composé de formule (I) telle que définie ci-dessus, ou son sel pharmaceutiquement acceptable, est administré ou administrable sous la forme d'une poudre, de cachets, de gélules ou de sachets.

De préférence, dans le cadre (i) du composé de formule (I) définie ci-dessus, ou d'un sel pharmaceutiquement acceptable de celui-ci, pour son utilisation dans la prévention ou le traitement du syndrome de sevrage alcoolique comme cela est défini ci-dessus, (ii) de l'utilisation dans la méthode de traitement définie ci-dessus, (iii) des produits définis ci-dessus, ou (iv) de la composition pharmaceutique définie ci-dessus, le composé de formule (I) définie ci-dessus, ou son sel pharmaceutiquement acceptable, est associé à au moins un composé additionnel destiné à la prévention ou au traitement du syndrome de sevrage alcoolique.

Ce composé additionnel est de préférence sélectionné dans le groupe constitué du méprobamate, du tétrabamate, d'un barbiturique, d'un neuroleptique, du clométhiazole, de l'acamprosate, d'un béta-bloquant, de la clonidine, de thiamine, du sulfate de magnésium, de la naltrexone, du disulfirame et de pyridoxine.

En outre, dans le cas de la composition pharmaceutique telle que définie ci-dessus, il est plus particulièrement préféré que le composé additionnel tel que défini ci-dessus soit sélectionné dans le groupe constitué du tétrabamate, du clométhiazole, de l'acamprosate, d'un béta-bloquant, de la clonidine, de thiamine, du sulfate de magnésium, de la naltrexone, du disulfirame, de pyridoxine et éventuellement du méprobamate.

Par ailleurs, en ce qui concerne (i) le composé de formule (I) définie ci-dessus, ou un sel pharmaceutiquement acceptable de celui-ci, pour son utilisation dans la prévention ou le traitement du syndrome de sevrage alcoolique comme cela est défini ci-dessus, (ii) l'utilisation dans la méthode de traitement définie ci-dessus, ou (iii) les produits définis ci-dessus, le groupe de composés additionnel défini ci-dessus comprend également les benzodiazépines (BZD), à savoir, de préférence, le diazépam, l'oxazépam, le lorazépam, l'alprazolam, le chlordiazépoxide, le clobazam, le prazépam, l'halazépam et le chlorazépate.

Avantageusement, l'association d'un composé de formule (I), ou d'un sel pharmaceutiquement acceptable de celui-ci, avec un composé additionnel destiné à la prévention ou au traitement du syndrome de sevrage alcoolique, permet notamment de diminuer la dose ou la durée d'administration dudit composé additionnel et ainsi de limiter ses effets secondaires.

### DESCRIPTION DES FIGURES

**Figure 1**
   Effets de l'étifoxine (EFX) ou de son véhicule (Véh) administrés 3h30 après le retrait de l'éthanol sur le score de convulsions chez la souris. Chaque valeur représente la moyenne ± esm. Le nombre d'animaux utilisés apparaît entre parenthèses. Le symbole étoile (*) représente p<0.05 par rapport aux contrôles alcoolisés.
**Figure 2**
   Effets du diazépam (DZP) ou de son véhicule (Véh) administrés 3h30 après le retrait de l'éthanol sur le score de convulsions chez la souris. Chaque valeur représente la moyenne ± esm. Le nombre d'animaux utilisés apparaît entre parenthèses. Le symbole étoile (*) représente p<0.05 par rapport aux contrôles alcoolisés.
**Figure 3**
   Effets de l'étifoxine (EFX), du diazépam (DZP) ou de leur véhicule respectif (Véh) sur l'aire sous la courbe calculée de 3 à 7h après le retrait de l'éthanol. Chaque valeur représente la moyenne ± esm. Le nombre d'animaux utilisés apparaît au pied des histogrammes. Le symbole dièse (#) représente p<0.05 par rapport aux contrôles alcoolisés.
**Figure 4**
   Effets de l'étifoxine (EFX; mg/kg), du diazépam (DZP; mg/kg) ou du véhicule (Véh) sur la latence d'entrée dans le compartiment clair chez la souris sevrée. Chaque valeur représente la moyenne ± esm. Le nombre d'animaux utilisés apparait au pied des histogrammes. * p<0.05 vs témoins non alcoolisés; # p<0.05 vs contrôles alcoolisés.
**Figure 5**
   Effets de l'étifoxine (EFX; mg/kg), du diazépam (DZP; mg/kg) ou du véhicule (Véh) sur le nombre de déplacements dans le compartiment clair chez la souris sevrée. Chaque valeur représente la moyenne ± esm. Le nombre d'animaux utilisés apparait au pied des histogrammes. * p<0.05 vs témoins non alcoolisés; # p<0.05 vs contrôles alcoolisés.
**Figure 6**
   Effets de l'étifoxine (EFX; mg/kg), du diazépam (DZP; mg/kg) ou du véhicule (Véh) sur le nombre de transition entre les 2 compartiments chez la souris sevrée. Chaque valeur représente la moyenne ± esm. Le nombre d'animaux utilisés apparait au pied des histogrammes. * p<0.05 vs témoins non alcoolisés; # p<0.05 vs contrôles alcoolisés.

### EXEMPLES

### MATERIEL ET METHODES

### 1. ANIMAUX

Des souris de souche NMRI (élevage Janvier), âgées de 5 à 6 semaines et de poids compris entre 25 et 30 g sont utilisées après 10 jours d'habituation aux conditions du laboratoire. Les conditions d'hébergement sont les suivantes : t° ambiante = 22 ± 2 °C ; hygrométrie relative = 50 ± 20 % ; cycle lumière/obscurité : 12 h/12 h (lumière de 7h00 à 19h00) ; nourriture A04 (SAFE, France) et boisson (eau du robinet) à volonté ; dix animaux groupés par cage de polypropylène (435x435x194 mm).

Cette étude avec des animaux de laboratoire a respecté les directives européennes en matière d'expérimentation animale (directive du 24/11/1986 ; 86/609/EC). Le laboratoire a pour n° d'agrément : B60-159-04 janvier 2005 (Ministère Français de l'agriculture).

### 2. PROTOCOLE D'ALCOOLISATION

Dans un premier temps, les animaux ont été familiarisés avec la nourriture liquide apportée sous un volume quotidien de 40 ml durant 7 à 10 jours. Celle-ci est constituée de lait chocolaté (Candia, France) auquel sont ajoutés 5 g/l d'un mélange de sels minéraux (ICN, France) et 3 g/l d'un mélange de vitamines (ICN, France).

Dans un deuxième temps, les animaux alcoolisés ont reçu la nourriture liquide contenant 3 % (p/v) d'éthanol (95°) durant 8 jours puis 4 % (p/v) d'éthanol (95°) durant 7 jours, tandis que les animaux témoins (non alcoolisés) ont eu accès à une nourriture avec l'équivalent calorique de dextrose (D-glucose, VWR, France).

Aucune nourriture solide, ni eau de boisson n'ont été proposées aux animaux durant cette période d'alcoolisation. Au terme des 15 jours d'alcoolisation (J15), le biberon avec l'alcool a été remplacé par un biberon de nourriture identique à celui des animaux témoins.

### 3. MESURE DE L'ALCOOLEMIE

Au cours d'une expérience séparée, des prélèvements de sang (300 µl) ont été effectués par ponction cardiaque chez des animaux anesthésiés à l'isoflurane après avoir été soumis au même protocole d'alcoolisation que celui décrit précédemment. Les prélèvements ont été réalisés à J7 et à J14 à 8h00 (terme de la phase active de l'animal) ou à 17h00 (proche du terme de la phase inactive de l'animal).

La méthode enzymatique de Bucher et Redetzki (1951) Klin Wochenschr 29: 615-616 a été utilisée pour mesurer l'alcoolémie dans le sang.

### 4. MESURE DES SIGNES DE SEVRAGE ALCOOLIQUE : INTENSITE DES CONVULSIONS INDUITES PAR LA MANIPULATION

Cette mesure est effectuée selon le protocole décrit par Watson and Little (1995) J Pharmacol Exp Ther 272: 876-884. Brièvement, l'animal est soulevé par la queue et maintenu durant 30 secondes à 30 cm d'une lampe pourvue d'une ampoule de 60W puis subit une légère rotation (360°). Le comportement de chaque animal est évalué selon une échelle de cotation de 0 à 5, selon des critères figurant dans le **Tableau 1 :**

**Tableau 1 : Différents niveaux de cotation du comportement chez la souris selon Watson et Little (1995)**

| **Score** | **Signes comportementaux** |
|---|---|
| 0 | Ne bouge pas |
| 1 | Léger tremblement au moment du soulèvement et de la rotation |
| 2 | Tremblement continu plus sévère au moment du soulèvement et de la rotation |
| 3 | Spasme avec extension clonique des pattes avant lors du soulèvement |
| 4 | Signes précédents qui continuent après avoir posé l'animal sur le couvercle de la cage |
| 5 | Activité myoclonique spontanée et séquences précédentes |

La mesure est réalisée avant l'expérience (conditions basales), 24 heures avant le retrait de l'éthanol puis le jour du retrait toutes les heures de 1 à 7 heures après le retrait du biberon de nourriture (7h00 - 8h00 le matin).

Les mesures effectuées avant l'expérience et à J14 ont permis de confirmer l'absence de convulsions spontanées ou apparues au cours de l'alcoolisation.

L'étifoxine (6.25 - 100 mg/kg) et le diazépam (1 et 4 mg/kg) sont administrés par voie intrapéritonéale (ip) 3h30 après le retrait du biberon. Une étude préliminaire a montré que les signes de sevrage étaient présents à ce temps (score ≥ 2). Les animaux contrôles reçoivent un volume équivalent de liquide véhicule.

Un score (de 0 à 5) est attribué pour chaque animal à chaque temps (de 1 à 7 h).

### 5. MESURE DU NIVEAU D'ANXIETE : EPREUVE DE LA BOITE A 2 COMPARTIMENTS

Le protocole décrit par Crawley and Goodwin (1980) Pharmacol Biochem Behav 1980; 13: 167-170 et légèrement modifié a été utilisé. Cette épreuve, reposant sur les tendances agoraphobiques et photophobiques des rongeurs, est classiquement utilisée pour mesurer leur niveau de peur ou d'anxiété.

Le dispositif est constitué d'une boîte en plexiglas à 2 compartiments (OSYS, Orga System; France) : l'un (18 x 27 x 30 cm) sombre et pourvu d'un couvercle, est éclairé à l'aide d'une lampe rouge (60W ; 4 lux) tandis que le plus grand (27 x 27 x 30 cm), peint en blanc, est éclairé à l'aide d'une lampe de 60 W (400 lux). Une entrée (7.5 x 7.5 cm) située au centre de la cloison qui sépare les 2 compartiments, relie les 2 compartiments. Ceux-ci, équipés de cellules photoélectriques infra-rouges (4 dans le compartiment clair et 3 dans le compartiment sombre) reliées à un compteur digital, permettent la détection et la mesure des déplacements de l'animal. Celui-ci est déposé au centre du compartiment sombre face à l'ouverture. La latence d'entrée (en sec.) dans le compartiment clair, le nombre de déplacements dans le compartiment clair et le nombre de transition entre les 2 compartiments sombre et clair sont relevés au terme d'une épreuve de 5 min. La boîte est nettoyée avec de l'eau alcoolisée (10 % ; v/v) pour éliminer toute trace odorante entre chaque passage. L'épreuve est réalisée 8 h après le retrait de la nourriture alcoolisée. Ce délai correspond au temps optimal pour la mesure du niveau d'anxiété chez l'animal soumis au sevrage alcoolique (Watson et Little (1994) Psychopharmacology (Berl) 114: 329-336).

L'étifoxine (6.25 - 50 mg/kg) ou le diazépam (1 mg/kg) est administré par voie ip 30 min avant l'épreuve. Les animaux contrôles reçoivent un volume équivalent de liquide véhicule.

### 6. PRODUITS UTILISES

L'étifoxine (lots 219 et 350 ; Biocodex - France) est mis en suspension dans une solution saline (NaCl-0.9 % ; p/v) de tween 80 à 1% (v/v) tandis que le diazépam (Valium®) est solubilisé dans une solution saline. Le volume d'administration par voie ip est de 0.1 ml/10 g.

### 7. EXPRESSION DES RESULTATS ET ANALYSE STATISTIQUE

Les résultats sont exprimés par la valeur moyenne ± erreur standard à la moyenne (esm). Concernant la mesure de l'intensité des convulsions induites par la manipulation, l'analyse de variance (ANOVA) à 2 facteurs (temps x traitement) avec mesures répétées sur le facteur temps suivie de manière post-hoc par le test de Student Newman-Keuls, a été utilisée pour localiser les différences entre les groupes expérimentaux. Les résultats sont aussi exprimés pour chaque animal par le calcul de l'aire sous la courbe selon la méthode des trapèzes de 3 à 7 h après le retrait du biberon (procédure 25 du logiciel PCS/PHARM-v4.2 Murray et al. (1981) Pharmacol Biochem Behav 15: 135-140). Ils sont comparés à l'aide d'une ANOVA à un facteur (traitement) ou de l'épreuve non paramétrique de Kruskal-Wallis selon le cas suivie respectivement par le test de Dunnett ou de Dunn avec le groupe contrôles alcoolisés comme référence. Dans le cas de l'épreuve de la boîte à 2 compartiments, les valeurs ont été comparées à l'aide de l'épreuve non paramétrique de Kruskall-Wallis suivie de manière posthoc par le test de Dunn pour localiser les différences entre les groupes. Le seuil de significativité est fixé à P < 0.05 (logiciel SigmaStat. V3.5, SPSS, USA).

### EXEMPLE 1

### MESURE DE L'ALCOOLEMIE

Au cours de cette expérience (n = 3-4 souris/expérience répétée 2 fois), à J7 et à J14, pour une consommation quotidienne d'alcool variant de 24 à 30 g/kg, l'alcoolémie correspondante mesurée au terme de la phase active de l'animal (8 h du matin) est égale à 2.05 ± 0.04 g/l, tandis qu'elle oscille entre 0.5 et 1.1 g/l au terme de la phase inactive de l'animal (17 h). Cette alcoolémie correspond à une consommation diurne d'alcool comprise entre 3 et 4 g/kg.

### EXEMPLE 2

### MESURE DU SCORE DES CONVULSIONS INDUITES PAR LA MANIPULATION

Pour les 2 expériences comportementales, la consommation journalière d'alcool à été de 28.9 ± 0.7 g/kg sans différence statistiquement significative entre les groupes.

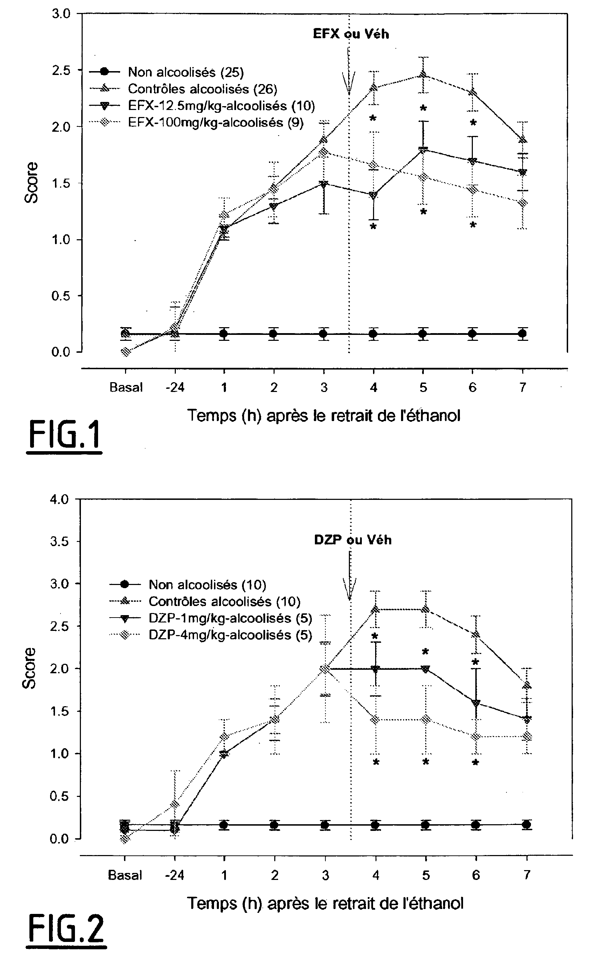

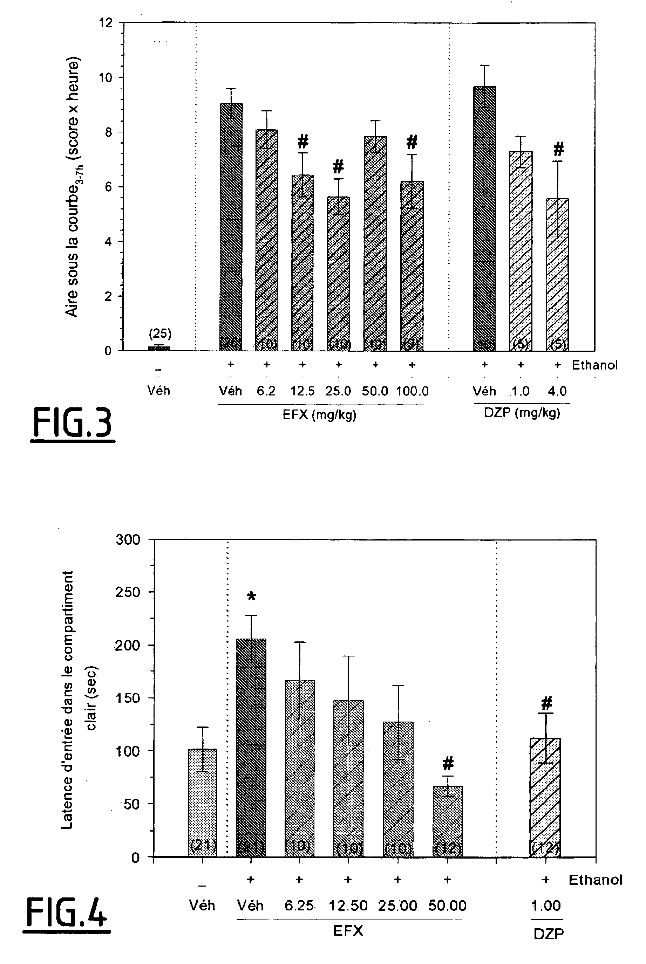

Les résultats concernant l'intensité des convulsions apparaissent dans les **Figures 1 à 3****.**

Le score de convulsions des animaux contrôles passe de 0 à 2.5 entre 4 et 6 h après retrait de l'éthanol. L'étifoxine aux doses de 12.5, 25 et 100 mg/kg réduit significativement le score de convulsions des animaux sevrés durant la même période. Le diazépam, principalement à la dose de 4 mg/kg, produit le même effet.

### EXEMPLE 3

### MESURE DU NIVEAU D'ANXIETE AU COURS DE L'EPREUVE DE LA BOITE A 2 COMPARTIMENTS

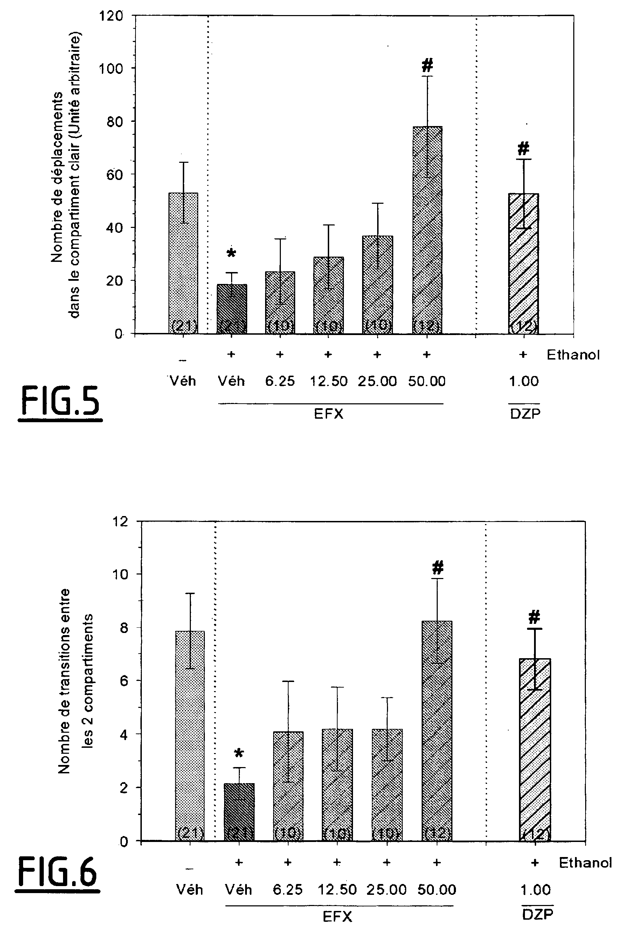

Les résultats sont montrés dans les **Figure 4 à 6****.**

Les animaux sevrés à l'éthanol comparés aux animaux témoins présentent une latence d'entrée dans le compartiment clair significativement plus élevée **(****Figure 4****),** un nombre de déplacements dans ce même compartiment significativement plus bas (**Figure 5**) et un nombre de transitions entre les 2 compartiments également significativement moins élevé (**Figure 6**).

L'étifoxine s'oppose aux effets du sevrage à l'éthanol sur ces 3 paramètres avec un effet statistiquement significatif à la dose de 50 mg/kg. A la dose de 1 mg/kg, le diazépam atténue également de manière significative les effets du retrait de l'éthanol sur ces mêmes paramètres.

## Revendications

1. Composé de formule (I) suivante : 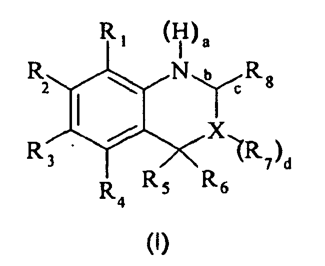 dans laquelle :
- a représente 0 ou 1 ;
- b représente une simple liaison ou une double liaison ;
- c représente une simple liaison ou une double liaison ;
- d représente 0 ou 1 ;
- X représente un atome d'oxygène ou d'azote, sous réserve que lorsque X représente un atome d'oxygène alors d vaut 0 et que lorsque X représente un atome d'azote alors d vaut 1 ;
- R₁, R₂, R₃, et R₄, identiques ou différents, représentent un atome d'hydrogène, un atome d'halogène, notamment choisi parmi F, CI, Br, ou I, un groupe hydroxyle, ou un groupe alkoxyle de 1 ou 2 atomes de carbone ;
- R₅ et R₆, identiques ou différents, représentent un atome d'hydrogène, un groupe alkyle ou cycloalkyle de 1 à 6 atomes de carbone, ou un groupe aryle de 6 atomes de carbone dont le noyau aromatique est éventuellement substitué par un ou plusieurs atomes d'halogène ou un ou plusieurs groupes hydroxyle, alkoxyle de 1 ou 2 atomes de carbone, trifluorométhyle ou nitro ;
- R₇ représente un atome d'hydrogène, un groupe hydroxyle, ou un groupe alkyle ou hydroxyalkyle de 1 à 3 atomes de carbone ;
- R₈ représente un atome d'oxygène ou un groupe -NR₉R₁₀, **R₉** et R₁₀, identiques ou différents, représentant un atome d'hydrogène, un groupe hydroxyle, ou un groupe alkyle ou hydroxyalkyle de 1 à 3 atomes de carbone, sous réserve que lorsque R₈ représente un atome d'oxygène alors a vaut 1, b représente une simple liaison et c représente une double liaison et que lorsque R₈ représente un groupe -NR₉R₁₀ alors a vaut 0, b représente une double liaison et c représente une simple liaison ;
ou sel pharmaceutiquement acceptable de celui-ci,
pour son utilisation dans la prévention ou le traitement du syndrome de sevrage alcoolique.

2. Composé ou sel pharmaceutiquement acceptable de celui-ci pour son utilisation selon la revendication 1, dans lequel la formule (I) est représentée par la formule (II) suivante : 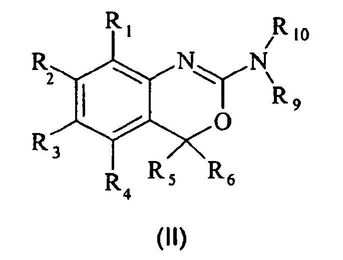 dans laquelle R₁, R₂, R₃, R₄, R₅, R₆, R₉ et R₁₀ sont tels que définis ci-dessus.

3. Composé ou sel pharmaceutiquement acceptable de celui-ci pour son utilisation selon la revendication 1 ou 2, de formules (III) et (IV) suivantes : 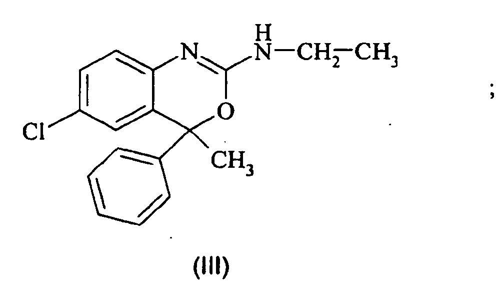 HCl

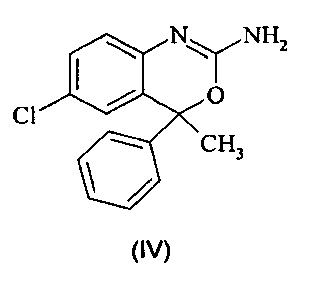

4. Composé ou sel pharmaceutiquement acceptable de celui-ci pour son utilisation selon la revendication 1, dans lequel la formule (I) est représentée par la formule (V) suivante : 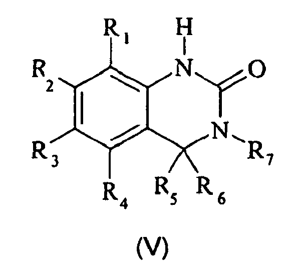 dans laquelle R₁, R₂, R₃, R₅, R₆, et R₇ sont tels que définis dans la revendication 1.

5. Composé ou sel pharmaceutiquement acceptable de celui-ci pour son utilisation selon la revendication 1 ou 4, de formules (VI) et (VII) suivantes :

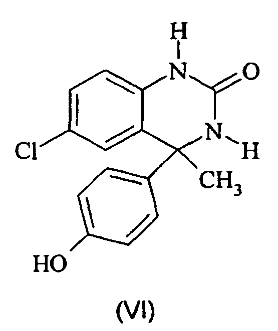

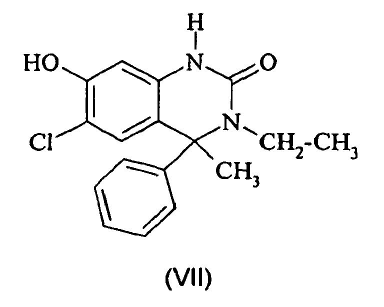

6. Composé ou sel pharmaceutiquement acceptable de celui-ci pour son utilisation selon l'une des revendications 1 à 5, pour une administration à une dose unitaire de 50 mg à 1500 mg.

7. Composé ou sel pharmaceutiquement acceptable de celui-ci pour son utilisation selon l'une des revendications 1 à 6, pour une administration avec un régime de dosage de 50 mg/j à 1500 mg/j.

8. Composé ou sel pharmaceutiquement acceptable de celui-ci pour son utilisation selon l'une des revendications 1 à 7, sous une forme convenant pour une administration par voie orale.

9. Composé ou sel pharmaceutiquement acceptable de celui-ci pour son utilisation selon l'une des revendications 1 à 8, sous forme d'une poudre, de cachets, de gélules ou de sachets.

10. Composé ou sel pharmaceutiquement acceptable de celui-ci pour son utilisation selon l'une des revendications 1 à 9, associé à au moins un composé additionnel destiné à la prévention ou au traitement du syndrome de sevrage alcoolique.

11. Composé ou sel pharmaceutiquement acceptable de celui-ci pour son utilisation selon la revendication 10, dans lequel le composé additionnel est sélectionné dans le groupe constitué du méprobamate, du tétrabamate, d'un barbiturique, d'un neuroleptique, du clométhiazole, de l'acamprosate, d'un béta-bloquant, de la clonidine, de thiamine, de pyridoxine, et d'une benzodiazépine.

12. Composé ou sel pharmaceutiquement acceptable de celui-ci selon l'une des revendications 1 à 11, pour son utilisation dans la prévention ou le traitement des convulsions, et éventuellement de l'anxiété, liées à un sevrage alcoolique.

13. Composition pharmaceutique, comprenant à titre de substances actives, au moins un composé de formule (I) tel que défini dans l'une des revendications 1 à 5, ou un sel pharmaceutiquement acceptable de celui-ci, et au moins un composé additionnel sélectionné dans le groupe constitué du tétrabamate, du clométhiazole, de l'acamprosate, d'un béta-bloquant, de la clonidine, de thiamine, et de pyridoxine, en association avec un véhicule pharmaceutiquement acceptable.

14. Produits contenant :
- au moins un composé de formule (I) tel que défini dans l'une des revendications 1 à 5, ou un sel pharmaceutiquement acceptable de celui-ci, et
- au moins un composé additionnel tel que défini dans la revendication 10 ou 11, comme produit de combinaison pour une utilisation simultanée, séparée ou étalée dans le temps pour la prévention ou le traitement du syndrome de sevrage alcoolique.

## Patentansprüche

1. Verbindung mit folgender Formel (I) : 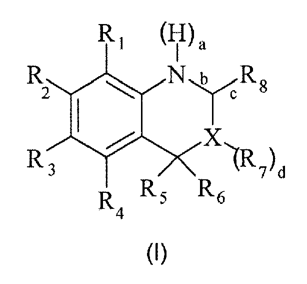 in der :
- a 0 oder 1 darstellt;
- b eine Einfachbindung oder eine Doppelbindung darstellt;
- c eine Einfachbindung oder eine Doppelbindung darstellt;
- d 0 oder 1 darstellt;
- X ein Sauerstoff- oder Stickstoffatom darstellt, mit der Maßgabe, dass, wenn X ein Sauerstoffatom darstellt, d dann 0 ist und, wenn X ein Stickstoffatom darstellt, d dann 1 ist;
- R₁, R₂, R₃, et R₄, die gleich oder unterschiedlich sind, ein Wasserstoffatom, ein Halogenatom, insbesondere ausgewählt aus F, Cl, Br und I, oder eine Hydroxylgruppe oder eine Alkoxylgruppe mit 1 oder 2 Kohlenstoffatomen darstellen;
- R₅ et R₆, die gleich oder unterschiedlich sind, ein Wasserstoffatom, eine Alkyl- oder Cycloalkylgruppe mit 1 bis 6 Kohlenstoffatomen oder eine Arylgruppe mit 6 Kohlenstoffatomen, deren aromatischer Ring gegebenenfalls mit einem oder mehreren Halogenatomen oder einer oder mehreren Hydroxylgruppe(n), Alkoxylgruppe(n) mit 1 oder 2 Kohlenstoffatomen, Trifluormethyl oder Nitro substituiert ist, darstellen;
- R₇ ein Wasserstoffatom, eine Hydroxylgruppe oder eine Alkyl- oder Hydroxyalkylgruppe mit 1 bis 3 Kohlenstoffatomen darstellt;
- R₈ ein Sauerstoffatom oder eine Gruppe -NR₉R₁₀ darstellt, wobei R₉ und R₁₀, die gleich oder unterschiedlich sind, ein Wasserstoffatom, eine Hydroxylgruppe oder eine Alkyl- oder Hydroxyalkylgruppe mit 1 bis 3 Kohlenstoffatomen darstellen, mit der Maßgabe dass, wenn R₈ ein Sauerstoffatom darstellt, dann a 1 ist, b eine Einfachbindung darstellt und c eine Doppelbindung darstellt, und dass, wenn R₈ eine Gruppe -NR₉R₁₀ darstellt, dann a 0 ist, b eine Doppelbindung darstellt und c eine Einfachbindung darstellt;
oder ein pharmazeutisch verträgliches Salz davon,
zu deren Verwendung bei der Prävention oder Behandlung des Alkoholentzugssyndroms

2. Verbindung oder ein pharmazeutisch verträgliches Salz davon zur Verwendung nach Anspruch 1, wobei die Formel (I) durch folgende Formel (II) dargestellt ist: 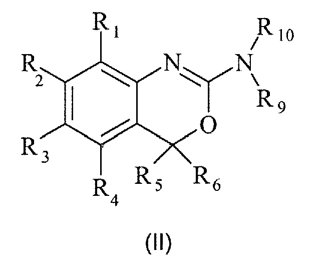 in welcher R₁, R₂, R₃, R₄, R₅, R₆, R₉ und R₁₀ wie oben definiert sind.

3. Verbindung oder ein pharmazeutisch verträgliches Salz davon zur Verwendung nach Anspruch 1 oder 2, mit folgenden Formeln (III) und (IV) 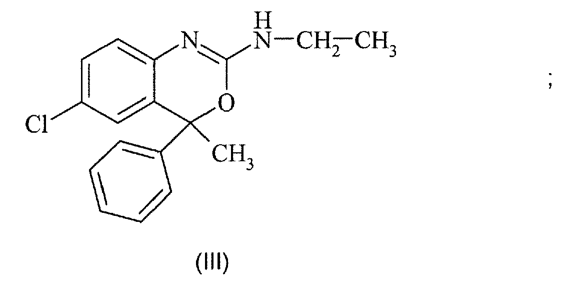 HCl

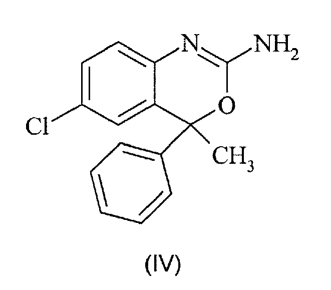

4. Verbindung oder ein pharmazeutisch verträgliches Salz davon zur Verwendung nach Anspruch 1, in der die Formel (I) durch folgende Formel (V) dargestellt ist: 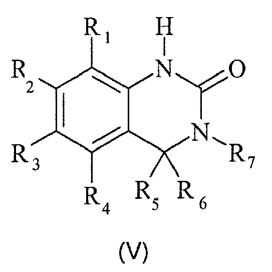 in welcher R₁, R₂, R₃, R₅, R₆, und R₇ wie im Anspruch 1 definiert sind.

5. Verbindung oder ein pharmazeutisch verträgliches Salz davon zur Verwendung nach Anspruch 1 oder 2, mit folgenden Formeln (VI) und (VII):

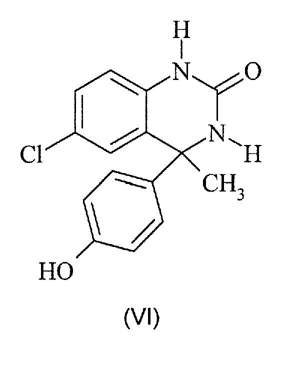

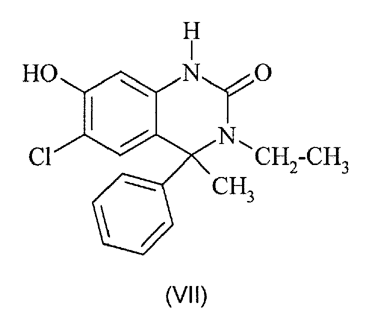

6. Verbindung oder ein pharmazeutisch verträgliches Salz davon zur Verwendung nach einem der Ansprüche 1 bis 5 für eine Verabreichung mit einer Einheitsdosis von 50 mg bis 1500 mg.

7. Verbindung oder ein pharmazeutisch verträgliches Salz davon zur Verwendung nach einem der Ansprüche 1 bis 6 für eine Verabreichung mit einem Dosierungsplan von 50 mg pro Tag bis 1500 mg pro Tag

8. Verbindung oder ein pharmazeutisch verträgliches Salz davon zur Verwendung nach einem der Ansprüche 1 bis 7 in einer Form, die für eine orale Verabreichung geeignet ist.

9. Verbindung oder ein pharmazeutisch verträgliches Salz davon zur Verwendung nach einem der Ansprüche 1 bis 8 in Form eines Pulvers, von Tabletten, Kapseln oder Beuteln.

10. Verbindung oder ein pharmazeutisch verträgliches Salz davon zur Verwendung nach einem der Ansprüche 1 bis 9, die mit wenigstens einer zusätzlichen Verbindung kombiniert ist, die zur Prävention oder Behandlung des Alkoholentzugssyndroms bestimmt ist.

11. Verbindung oder ein pharmazeutisch verträgliches Salz davon zur Verwendung nach Anspruch 10, in welcher die zusätzliche Verbindung ausgewählt ist aus der Gruppe bestehend aus Meprobamat, Tetrabamat, einem Barbiturat, einem Neuroleptikum, Clomethiazol, Acamprosat, einem Beta-Blocker, Clonidin, Thiamin, Pyridoxin und einem Benzodiazepin.

12. Verbindung oder ein pharmazeutisch verträgliches Salz davon nach einem der Ansprüche 1 bis 11 zur Verwendung in der Prävention oder Behandlung von Konvulsionen und gegebenenfalls der Angst, die mit einem Alkoholentzug verbunden sind.

13. Pharmazeutische Zusammensetzung, umfassend als aktive Substanz wenigstens eine Verbindung der Formel (I), wie sie in einem der Ansprüche 1 bis 5 definiert ist, oder ein pharmazeutisch verträgliches Salz davon und wenigstens eine zusätzliche Verbindung, die ausgewählt ist aus der Gruppe bestehend aus Tetrabamat, Clomethiazol, Acamprosat, einem Beta-Blocker, Clonidin, Thiamin und Pyridoxin in Verbindung mit einem pharmazeutisch verträglichen Vehikel.

14. Produkte, enthaltend:
- wenigstens eine Verbindung der Formel (I), wie sie in einem der Ansprüche 1 bis 5 definiert ist, oder ein pharmazeutisch verträgliches Salz davon; und
- wenigstens eine zusätzliche Verbindung, wie im Anspruch 10 oder 11 definiert,
als Kombinationsprodukt für eine gleichzeitige, getrennte oder über die Zeit verteilte Verwendung zur Prävention oder Behandlung des Alkoholentzugssyndroms.

## Claims

1. A compound having the following formula (I): 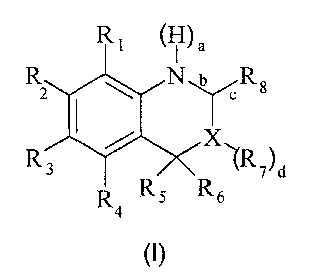 in which:
- a represents 0 or 1,
- b represents a single bond or a double bond,
- c represents a single bond or a double bond,
- d represents 0 or 1,
- X represents an oxygen or nitrogen atom, provided that when X represents an oxygen atom then d has the value of 0 and when X represents a nitrogen atom then d has the value of 1,
- R₁, R₂, R₃, and R₄, which are the same or different, represent a hydrogen atom, a halogen atom, in particular selected from F, Cl, Br, or I, a hydroxyl group, or an alkoxyl group having 1 or 2 carbon atoms,
- R₅ and R₆, which are the same or different, represent a hydrogen atom, an alkyl or cycloalkyl group having from 1 to 6 carbon atoms, or an aryl group having 6 carbon atoms in which the aromatic ring may be substituted by one or more halogen atoms or one or more hydroxyl, alkoxyl having 1 or 2 carbon atoms, trifluoromethyl or nitro groups,
- R₇ represents a hydrogen atom, a hydroxyl group, or an alkyl or hydroxyalkyl group having from 1 to 3 carbon atoms,
- R₈ represents an oxygen atom or a -NR₉R₁₀ group, R₉ and R₁₀, which are the same or different, representing a hydrogen atom, a hydroxyl group, or an alkyl or hydroxyalkyl group having from 1 to 3 carbon atoms, provided that when R₈ represents an oxygen atom then a has the value 1, b represents a single bond and c represents a double bond and that when R₈ represents a -NR₉R₁₀ group then a has the value 0, b represents a double bond and c represents a single bond;
or a pharmaceutically acceptable salt thereof,
for use in the prevention or treatment of alcohol withdrawal syndrome.

2. The compound or pharmaceutically acceptable salt thereof for use according to claim 1, wherein formula (I) is represented by the following formula (II): 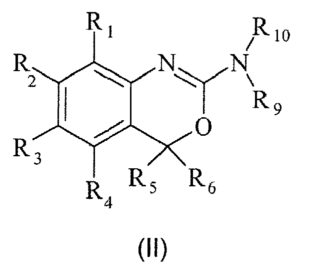 in which R₁, R₂, R₃, R₄, R₅, R₆, R₉ and R₁₀ are as defined above.

3. The compound or pharmaceutically acceptable salt thereof for use according to claim 1 or 2, of the following formulae (III) and (IV): 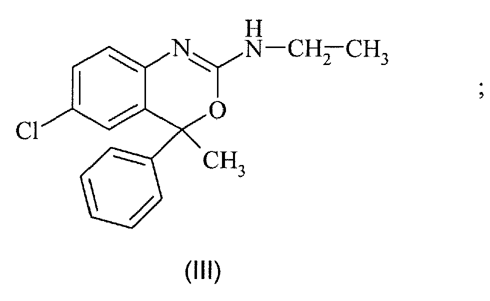 HCl

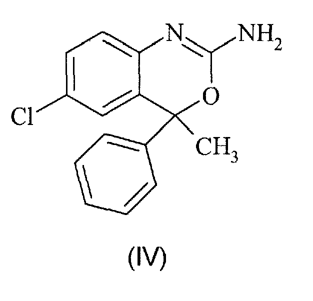

4. The compound or pharmaceutically acceptable salt thereof for use according to claim 1, wherein formula (I) is represented by the following formula (V): 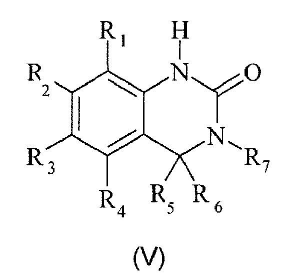 in which R₁, R₂, R₃, R₅, R₆, and R₇ are as defined in claim 1.

5. The compound or pharmaceutically acceptable salt thereof for use according to claim 1 or 4, of the following formulae (VI) and (VII):

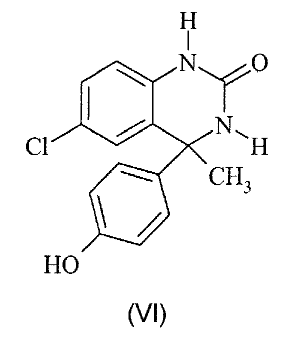

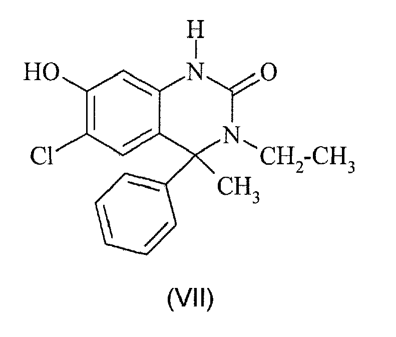

6. The compound or pharmaceutically acceptable salt thereof for use according to any of claims 1 to 5, for administration at a unit dose of from 50 mg to 1500 mg.

7. The compound or pharmaceutically acceptable salt thereof for use according to any of claims 1 to 6, for administration with a dosage regimen of from 50 mg/d to 1500 mg/d.

8. The compound or pharmaceutically acceptable salt thereof for use according to any of claims 1 to 7, in a form suitable for oral administration.

9. The compound or pharmaceutically acceptable salt thereof for use according to any of claims 1 to 8, in the form of a powder, tablets, capsules or sachets.

10. The compound or pharmaceutically acceptable salt thereof for use according to any of claims 1 to 9, associated to at least one additional compound intended for the prevention or treatment of alcohol withdrawal syndrome.

11. The compound or pharmaceutically acceptable salt thereof for use according to claim 10, wherein the additional compound is selected from the group consisting of meprobamate, tetrabamate, a barbiturate, a neuroleptic, clomethiazole, acamprosate, a beta-blocker, clonidine, thiamine, pyridoxine, and a benzodiazepine.

12. The compound or pharmaceutically acceptable salt thereof according to any of claims 1 to 11, for use in the prevention or treatment of convulsions, and optionally of anxiety, linked to alcohol withdrawal.

13. A pharmaceutical composition, comprising as active substances, at least one compound of formula (I) as defined in any of claims 1 to 5, or a pharmaceutically acceptable salt thereof, and at least one additional compound selected from the group consisting of tetrabamate, clomethiazole, acamprosate, a beta-blocker, clonidine, thiamine, and pyridoxine, in association with a pharmaceutically acceptable carrier.

14. Products comprising:
• at least one compound of formula (I) as defined in any of claims 1 to 5, or a pharmaceutically acceptable salt thereof, and
• at least one additional compound as defined in claim 10 or 11,
as a combined preparation for simultaneous, separate or sequential use in the prevention or treatment of alcohol withdrawal syndrome.
